# EUROPEAN PATENT APPLICATION

(11) **EP 4 810 507 A1**
(43) Date of publication of application: **23.09.2026**
(21) Application number: 25165135.2
(22) Date of filing: 20.03.2025
(51) Int. Cl.: C07H 1/00, C07H 21/02, C07H 21/04

(54) **PROCESS FOR THE REMOVAL OF A MONOMETHOXYTRITYL PROTECTING GROUP FROM A MONOMETHOXYTRITYL PROTECTED AMINO LINKER MOIETY ATTACHED TO AN OLIGONUCLEOTIDE**

(71) Applicant: F. Hoffmann-La Roche AG, 4070 Basel (CH)
(72) Inventor: The designation of the inventor has not yet been filed
(74) Representative: Rauber, Beat

(57) **Abstract**

The invention relates to a novel process for the removal of a monomethoxytrityl protecting group from a monomethoxytrityl protected amino linker moiety attached to the 5' or 3'-terminus of an oligonucleotide.

## Description

Process for the removal of a monomethoxytrityl protecting group from a monomethoxytrityl protected amino linker moiety attached to the 5' or 3'-terminus of an oligonucleotide,
comprising,
a) providing an aqueous ammonia solution obtained from the cleaving off the oligonucleotide from the resin;
b) evaporating the solution, while removing ammonia until the pH of the reaction mixture is in the range of pH 6.0 to pH 9.0;
c) removing the deprotection by-products,
   and optionally
d) isolation and purification.

The oligonucleotide synthesis in principle is a stepwise addition of nucleoside residues to the 5'-terminus of the growing chain until the desired sequence is assembled.

As a rule, each addition is referred to as a synthetic cycle and in principle consists of the chemical reactions
a₁) de-blocking the 5' protected hydroxyl group on the solid support,
a₂) coupling the first nucleoside as activated phosphoramidite with the free hydroxyl group on the solid support,
a₃) oxidizing or sulfurizing the respective P-linked nucleoside to form the respective phosphodiester (P=O) or the respective phosphorothioate (P=S);
a₄) optionally, capping any unreacted hydroxyl groups on the solid support,
a₅) de-blocking the 5' hydroxyl group of the first nucleoside attached to the solid support;
a₆) coupling the second nucleoside as activated phosphoramidite to form the respective P-O linked dimer;
a₇) oxidizing or sulfurizing the respective P-O linked dinucleoside to form the respective phosphodiester (P=O) or the respective phosphorothioate (P=S);
a₈) optionally, capping any unreacted 5' hydroxyl groups;
a₉) repeating the previous steps as to as until the desired sequence is assembled,

The reaction sequence may alternatively start with de-blocking of the 5' protected hydroxyl group of the nucleoside which is preloaded on the solid support. The subsequent steps follow the sequence as outlined above.

Finally, the assembled oligonucleotide is subjected to deblocking conditions, backbone deprotection using amine reagents and is then cleaved from the solid support and subsequent downstream processing and purification methods provide the desired pure oligonucleotide.

In a variant of oligonucleotide synthesis the hydroxy protecting group, monomethoxytrityl (MMT), is left on the 5' end after the assembly of the oligonucleotide chain. This approach allows for further purification of the oligonucleotide after cleavage from the solid support. The MMT group's hydrophobic nature enables selective retention of full-length sequences during purification steps. The final removal of the MMT group is typically achieved through an acidic treatment, often using acetic acid or acetic acid derivatives (e.g. trifluoroacetic acid (TFA)).

A method for the removal of the monomethoxytrityl protecting group from a monomethoxytrityl protected amino linker moiety has been described in the International Patent Publication WO2016/137923. The method comprises
a) providing an aqueous solution of the oligomeric compounds having monomethoxytrityl protected amino groups;
b) heating the solution to from about 35 °C to about 45°C;
c) adjusting the pH of the heated solution to from about 4.0 to about 5.5 by addition of an acid with mixing; and
d) mixing the acidified solution while maintaining the pH at from about 4.0 to about 5.5 and the temperature at from about 35 °C to about 45°C until essentially all of the monomethoxytrityl groups are removed thereby deprotecting the oligomeric compounds.

Object of the present invention was to find an alternative process for the removal of a monomethoxytrityl protecting group from a monomethoxytrityl protected amino linker moiety which can be run under milder conditions.

It was found that the object could be achieved with a novel process for the removal of a monomethoxytrityl protecting group from a monomethoxytrityl protected amino linker moiety linked to either the 5' or 3'-terminus comprising,
a) providing an aqueous ammonia solution obtained from the cleaving off the oligonucleotide from the resin;
b) evaporating the solution, while removing ammonia until the pH of the reaction mixture is in the range of pH 6.0 to pH 9.0;
c) removing the deprotection by-products
   and optionally
d) isolation and purification.

The following definitions are set forth to illustrate and define the meaning and scope of the various terms used to describe the invention herein.

The term monomethoxytrityl protected amino linker moiety typically refers to a monomethoxytrityl protected amino linker attached to the 5' or 3'-terminus of an oligonucleotide. Typically the amino linker is a C₂₋₁₂-alkyl-linker or an ethylene glycol linker containing 1 to 10 ethylene glycol units, both of which carry a monomethoxytrityl protected amino group covalently bound to the linker. Preferred are C₃₋₈-alkyl-linkers. Preferred ethylene glycol linkers contain 2 to 6 ethylene glycol units. As a rule the amino linkers are introduced via commercially available monomethoxytrityl protected linker phosphoroamidites under standard oligonucleotide synthesis conditions.

The term oligonucleotide as used herein is defined as it is generally understood by the skilled person as a molecule comprising two or more covalently linked nucleotides. For use as a therapeutically valuable oligonucleotide, oligonucleotides are typically synthesized as 10 to 40 nucleotides, preferably 10 to 25 nucleotides in length.

The oligonucleotides may consist of optionally modified DNA, RNA or LNA nucleoside monomers or combinations thereof.

The LNA nucleoside monomers are modified nucleosides which comprise a linker group or a bridge between C2' and C4' of the ribose sugar ring of a nucleotide. These nucleosides are also termed bridged nucleic acid or bicyclic nucleic acid (BNA) in the literature.

Optionally modified as used herein refers to nucleosides modified as compared to the equivalent DNA, RNA or LNA nucleoside by the introduction of one or more modifications of the sugar moiety or the nucleobase moiety. In a preferred embodiment the modified nucleoside comprises a modified sugar moiety, and may for example comprise one or more 2' substituted nucleosides and/or one or more LNA nucleosides. The term modified nucleoside may also be used herein interchangeably with the term "nucleoside analogue" or modified "units" or modified "monomers".

The DNA, RNA or LNA nucleosides are as a rule linked by a phosphodiester (P=O) and / or a phosphorothioate (P=S) internucleoside linkage which covalently couples two nucleosides together.

Accordingly, in some oligonucleotides all internucleoside linkages may consist of a phosphodiester (P=O), in other oligonucleotides all internucleoside linkages may consist of a phosphorothioate (P=S) or in still other oligonucleotides the sequence of internucleoside linkages vary and comprise both phosphodiester (P=O) and phosphorothioate (P=S) internucleosides.

The nucleobase moieties are indicated by the letter code A (adenine), C (cytosine), T (thymine), G (guanine) or U (uracil). Each letter code may optionally include modified nucleobases of equivalent function. For example the letter code E stands for the modified cytosine 5-methyl cytosine. Modified nucleobases include, but are not limited to nucleobases carrying protecting groups such as tert-butylphenoxyacetyl, phenoxyacetyl, benzoyl, acetyl, isobutyryl or dimethylformamidino.

The described principles of the oligonucleotide synthesis are well known in the art (see Wikipedia contributors. "Oligonucleotide synthesis" Wikipedia, The Free Encyclopedia., 19 Jan. 2021. Web. 16 Feb. 2021).

Larger scale oligonucleotide synthesis nowadays is carried out automatically using computer controlled synthesizers.

As a rule, oligonucleotide synthesis is a solid-phase synthesis, wherein the oligonucleotide being assembled is covalently bound, via its 3'-terminal hydroxy group, to a solid support material and remains attached to it over the entire course of the chain assembly. Suitable supports are the commercially available macroporous polystyrene supports like the Primer support 5G from GE Healthcare or the NittoPhase^{®}HL support from Kinovate, or controlled pore glass supports like the nucleobase pre-loaded support from LGC.

As outlined above the oligonucleotide synthesis in principle is a stepwise addition of nucleoside residues to the 5'-terminus of the growing chain until the desired sequence is assembled.

The subsequent cleavage from the resin can be performed with concentrated aqueous ammonia. Additional reagents, such as lower aliphatic alcohols like ethanol or isopropanol, can be added to the deprotection mixture to facilitate mild cleavage. Within this cleavage procedure also the protecting groups on the (thio)phosphate backbone and the nucleobase are removed.

As outlined above the process of the present invention relates to a process for the removal of a monomethoxytrityl protecting group from a monomethoxytrityl protected amino linker moiety linked to either the 5' or 3'-terminus comprising,
a) providing an aqueous ammonia solution obtained from the cleaving off the oligonucleotide from the resin;
b) evaporating the solution, while removing ammonia until the pH of the reaction mixture is in the range of pH 6.0 to pH 9.0;
c) removing the deprotection by-products
   and optionally
d) isolation and purification.

In a preferred aspect of the invention the monomethoxytrityl protected amino group is linked to the 5' terminus.

The evaporation is typically performed at a temperature of 30°C to 70°C, preferably at 30°C to 60°C, more preferably at about 40°C.

Further the evaporation can be run under reduced pressure or also at normal pressure. A pressure range of 5 mbar to 1 bar can usually be applied.

However, for the scenario described later, where an acid or acidic salt is added, a reduced pressure is preferable.

Preferably, the evaporation and therewith the removal of ammonia is continued until the pH of the reaction mixture is in the range of pH 6.0 to pH 8.0 and the target concentration reaches 250 OD/mL to 4000 OD/mL, more preferably 1000 OD/mL to 3000 OD/mL.

In a preferred aspect of the present invention removing of the deprotection by-products in step c) comprises either
c1) extracting the reaction solution with organic solvents and subsequently feeding the solution to tangential flow filtration or
c2) directly feeding the reaction solution to tangential filtration.

Suitable organic solvents for the extraction in step c1) can be selected from ethyl acetate, 2-methyl tetrahydrofuran, tert. butyl methyl ether or cyclopentyl methyl ether. The addition of a lower aliphatic alcohol, such as methanol, ethanol or isopropanol may help to facilitate phase separation. The mixture ethyl acetate and isopropanol was found to be a preferred organic solvent for the extraction of the reaction mixture. Thereafter the solution is fed to tangential flow filtration.

Alternatively, according to step c2), the reaction solution can directly be fed to tangential flow filtration. A previous dilution of the reaction mixture with a suitable organic solvent, selected from acetonitrile, ethanol, n-propanol or i-propanol, water or mixtures thereof, was found to be advantageous. Preferred is a mixture of acetonitrile and water. The optimal ratio solvent and water depends on the solvent, but as a rule a 30% to 90% v/v solvent in water proved suitable.

Dependent from the deprotection procedure a salt exchange and a desalting step e.g. by tangential flow filtration may prepare the oligonucleotide for a subsequent conjugation step, for instance with a targeting cluster compound, such as with a GalNAc cluster compound. The conjugation typically is accomplished under standard peptide coupling conditions. The US Patent Publication US2020/0361983 can be taken as illustrative for such reaction. Classical purification and isolation technologies, such as ionexchange chromatography and lyophilization can render the pure oligo cluster conjugate.

In a preferred aspect of the invention, subsequent to the provision of the aqueous ammonia solution in step a), acetic acid or an acidic salt is added.

The acetic acid can be applied in the form of the glacial acetic acid or also as a diluted aqueous acetic acid.

The acidic salt can be selected from acetates, borates, chlorides, citrates, dihydrogencarbonates, dihydrogenphosphates, hydrogen sulfates, monohydrogenphosphates or sulfates with ammonium, potassium or sodium as their cation.

Preferred representatives of acidic salts are ammonium chloride and sodium dihydrogenphosphate.

Typically the acetic acid is added in an amount of 6.0 to 10.0 equivalents, preferably 7.0 to 8.5 equivalents, related to 1.0 equivalent of the oligonucleotide.

The amount of the acidic salts heavily depends on the type of salt and can vary in a broad range of 20 to 600 equivalents, related to 1.0 equivalent of the oligonucleotide

In a further preferred aspect of the invention the process is run with the addition of acetic acid under conditions where
a1) acetic acid is added until a pH in the range of pH 11 to 13 is reached;
b1) the reaction mixture is evaporated at a temperature of 30°C to 70°C, preferably at 30°C to 60°C, more preferably at about 40°C and at a pressure of 10 mbar to 100 mbar, preferably 40 mbar to 60 mbar, while removing ammonia until the pH of the solution is in the range of pH 6.0 to pH 8.0,
c11) the resulting reaction mixture is extracted with ethyl acetate and isopropanol and subsequently fed to tangential flow filtration or,
c21) diluted with acetonitrile and water and directly fed to tangential flow filtration and optionally
d1) subjected to isolation and further purification.

Still more preferred is the process wherein removing of the deprotection by-products is accomplished according to step c21).

It was found advantageous to stir the reaction mixture at normal pressure for 1 h to 7 h after the evaporation step b1) and before the treatment in step c) to further facilitate monomethoxytrityl group deprotection.

Preferably the tangential flow filtration involves a salt exchange by running the reaction solution against a sodium salt to effect a salt exchange from the ammonium salt to the sodium salt of the oligonucleotide. Suitable sodium salts can be selected from halogenides, sulfates, hydrogen carbonates or from carbonates, such as Na₂SO₄, NaCl, NaHCO₃, Na₂CO₃, NaBr or NaI. In addition the tangential flow filtration involves a desalting of the solution

Isolation can happen by concentration of the solution obtained from tangential flow filtration in vacuo. Further purification can be achieved via chromatography.

By way of illustration the oligonucleotide can be selected from:
**TGN.Lo.eAs.eTs.eEs.eEs.eEs.dAs.dEs.dGs.dEs.dEs.dEs.dEs.dTs.dGs.dTs.eEs.eEs.eA** s.eGs.eE.

The symbols have the following meaning:
Sequence information is provided from 5'-end (left) to 3'-end (right). Each nucleotide is described by three-letters: First letter: Sugar (d = 2'-deoxyribose / DNA, e = 2'-O-(2-methoxyethyl) ribose / MOE); Second letter: Nucleobase (A = adenine, E = 5-methylcytosine, G = guanine, L = hexylaminolinker , T = thymine); Third letter: Backbone (o = phosphate, s = thiophosphate); Conjugate: TGN = Tris GalNAc cluster.

The compounds disclosed herein have the following nucleobase sequences SEQ ID No. 1: atcccacgccccctgtccagc

### Examples

### Abbreviations:

| | |
|---|---|
| AcOH | acetic acid |
| Bz | benzyl |
| DCA | dichloroacetic acid |
| DCI | dicyanoimidazole |
| iBu | i-butyl |
| MOE | 2-methoxy ethyl |
| MMT | monomethoxytrityl |
| NMI | N-methylimidazole |
| OD | optical density |
| TFF system | tangential flow filtration system |
| TGN | Tris GalNAc cluster |

### 1. Oligonucleotide Synthesis

### TGN.Lo.eAs.eTs.eEs.eEs.eEs.dAs.dEs.dGs.dEs.dEs.dEs.dEs.dTs.dGs.dTs.eEs.eEs.eA s.eGs.eE

Sequence information is provided from 5'-end (left) to 3'-end (right). Each nucleotide is described by three-letters: First letter: Sugar (d = 2'-deoxyribose / DNA, e = 2'-O-(2-methoxyethyl) ribose / MOE); Second letter: Nucleobase (A = adenine, E = 5-methylcytosine, G = guanine, L = hexylaminolinker, T = thymine); Third letter: Backbone (o = phosphate, s = thiophosphate); Conjugate: TGN = Tris GalNAc cluster.

The title compound was produced by standard phosphoramidite chemistry on solid phase at a scale of 2.6 mmol using an AKTA Oligopilot 100 and Primer Support Unylinker (NittoPhase HL Unylinker 400).

The following phosphoramidites have been used in each cycle:

| **Cycle** | **P-amidite** | **Cycle** | **P-amidite** |
|---|---|---|---|
| 1 | eE(Bz) | 12 | dE(Bz) |
| 2 | eG(iBu) | 13 | dG(iBu) |
| 3 | eA(Bz) | 14 | dE(Bz) |
| 4 | eE(Bz) | 15 | dA(Bz) |
| 5 | eE(Bz) | 16 | eE(Bz) |
| 6 | dT | 17 | eE(Bz) |
| 7 | dG(iBu) | 18 | eE(Bz) |
| 8 | dT | 19 | eT |
| 9 | dE(Bz) | 20 | eA(Bz) |
| 10 | dE(Bz) | 21 | L(MMT) |
| 11 | dE(Bz) | | |

In general 1.4 equiv. of the phosphoramidites were employed. All reagents were used as received from commercially available sources and reagent solutions at the appropriate concentration were prepared (see details below). Cleavage and deprotection was achieved using a mixture of ammonium hydroxide and isopropanol (3/2; v/v) to give the crude MMT protected oligonucleotide: (MMT)
Lo.eAs.eTs.eEs.eEs.eEs.dAs.dEs.dGs.dEs.dEs.dEs.dEs.dTs.dGs.dTs.eEs.eEs.eAs.eGs.eE

### Standard Reagent Solutions

| | |
|---|---|
| Detritylation | DCA in toluene (1/9 v/v) |
| Phosphoramidites | 0.2 M in acetonitrile |
| Activator | 1.0 M DCI, 0.1 M NMI in acetonitrile |
| Sulfurization | 0.1 M xanthanhydride in pyridine/acetonitrile (7/3 v/v) |
| Cap A | NMI/pyridine/acetonitrile 20/30/50 (v/v/v) |
| Cap B | 20% acetic anhydride in acetonitrile (v/v) |
| Amine wash | 20% diethylamine in acetonitrile (v/v) |
| Cleavage and Deprotection | 28-32% aqueous ammonium hydroxide / isopropanol (3/2 v/v) |

### 2. MMT Deprotection procedures

### Example 1 (mild temperature):

The solution containing the crude MMT protected oligonucleotide is concentrated at 40 °C in vacuo (50 mbar) to achieve a pH of 8 and a concentration of 1800 OD/mL. The resulting cloudy mixture is stirred at 40 °C under normal pressure for 6 h to facilitate complete MMT deprotection. The reaction mixture (20 mL) is extracted with a mixture of ethyl acetate (20 mL) and isopropanol (1 mL) to remove the deprotection by-product. The solution is submitted to salt exchange followed by desalting using tangential flow filtration. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

### Example 2 (elevated temperature):

The solution containing the crude MMT protected oligonucleotide is concentrated at 60 °C in vacuo (50 mbar) to achieve a pH of 8 and a concentration of 2200 OD/mL. The resulting cloudy mixture is stirred at 60 °C under normal pressure for 1 h to facilitate complete MMT deprotection. The reaction mixture (20 mL) is extracted with a mixture of ethyl acetate (20 mL) and isopropanol (1 mL) to remove the deprotection by-product. The solution is submitted to salt exchange followed by desalting using tangential flow filtration. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

### Example 3 (NH₄Cl):

The solution containing the crude MMT protected oligonucleotide is concentrated at 40 °C in vacuo (50 mbar) to achieve a pH of 9 followed by addition of 100 mL 2M aqueous NH₄Cl. The resulting cloudy mixture (pH 7.5) is stirred at 40 °C under normal pressure for 2 h to facilitate complete MMT deprotection. The reaction mixture (130 mL) is extracted with a mixture of ethyl acetate (130 mL) and isopropanol (13 mL) to remove the deprotection by-product. The solution is submitted to salt exchange followed by desalting using tangential flow filtration. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

### Example 4 (NaH₂PO₄):

The solution containing the crude MMT protected oligonucleotide is concentrated at 40 °C in vacuo (50 mbar) to achieve a pH of 9 followed by addition of 9 mL 1M aqueous NaH₂PO₄. The resulting cloudy mixture (pH 7) is stirred at 40 °C under normal pressure for 3.5 h to facilitate complete MMT deprotection. The reaction mixture (40 mL) is extracted with a mixture of ethyl acetate (40 mL) and isopropanol (4 mL) to remove the deprotection by-product. The solution is submitted to salt exchange followed by desalting using tangential flow filtration. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

### Example 5 (AcOH):

The solution containing the crude MMT protected oligonucleotide is concentrated at 40 °C in vacuo (50 mbar) to achieve a pH of 9 followed by addition of 150 µL AcOH. The resulting cloudy mixture (pH 6.2) is stirred at 40 °C under normal pressure for 2 h to facilitate complete MMT deprotection. The reaction mixture (30 mL) is extracted with a mixture of ethyl acetate (30 mL) and isopropanol (3 mL) to remove the deprotection by-product. The solution is submitted to salt exchange followed by desalting using tangential flow filtration. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

### Example 6 (AcOH):

The solution containing the crude MMT protected oligonucleotide (2.6 mmol in 471.4 g solution, pH 12.1) is treated with glacial acetic acid (1.25 g, 20.8 mmol, 8.0 equiv.) resulting in a pH of 11.9. The mixture is concentrated at 40 °C in vacuo (50 mbar) to achieve a pH of 7.4 and a target concentration of approx. 2500 OD/mL. The resulting cloudy mixture is stirred at 40 °C under normal pressure for 2 h to facilitate complete MMT deprotection. The reaction mixture (148 g) is diluted with acetonitrile (157 g) and water (153 g) to obtain a clear yellow solution. The solution is charged to a TFF system (5 kDa, 2.0 bar TMP) and diafiltered against 15 volumes of sodium bromide followed by 15 volumes of water to facilitate removal of the deprotection by-product and salt exchange to the desired sodium salt. The deprotected oligonucleotide is obtained in a concentration of 450 OD/mL in 87.5% UV purity. The resulting oligonucleotide solution is concentrated using thin film evaporation in vacuo. This is followed by conjugation with a GalNAc cluster using standard amide coupling reagents. The crude conjugated oligonucleotide is purified by IEX chromatography using a salt gradient. The resulting solution is desalted using tangential flow filtration and the pure product is isolated by lyophilization.

## Claims

1. Process for the removal of a monomethoxytrityl protecting group from a monomethoxytrityl protected amino linker moiety attached to the 5' or 3'-terminus of an oligonucleotide,
comprising,
a) providing an aqueous ammonia solution obtained from the cleaving off the oligonucleotide from the resin,
b) evaporating the solution, while removing ammonia until the pH of the reaction mixture is in the range of pH 6.0 to pH 9.0,
c) removing the deprotection by-products
and optionally
d) isolation and purification.

2. Process of claim 1, wherein the monomethoxytrityl protected amino linker is linked to the 5' terminus of the oligonucleotide.

3. Process of claim 1 or 2, wherein the monomethoxytrityl protected amino linker is a monomethoxytrityl protected amino C₂₋₁₂-alkyl-linker or a monomethoxytrityl protected amino ethylene glycol linker containing 1 to 10 ethylene glycol units.

4. Process of any one of claims 1 to 3, wherein the evaporation is performed at a temperature of 30°C to 70°C.

5. Process of any one of claims 1 to 4, wherein the evaporation is performed at a pressure of 5 mbar to 1 bar.

6. Process of any one of claims 1 to 5, wherein the evaporation is performed until the pH of the reaction mixture is in the range of pH 6.0 to pH 8.0.

7. Process of any one of claims 1 to 6, wherein the evaporation is performed in a manner that a target concentration of 250 OD/mL to 4000 OD/mL is reached.

8. Process of any one of claims 1 to 7, wherein subsequent to the provision of the aqueous ammonia solution in step a) acetic acid or an acidic salt is added.

9. Process of claim 8, wherein the acidic salt can be selected from acetates, borates, chlorides, citrates, dihydrogen carbonates, dihydrogen phosphates, hydrogen sulfates, monohydrogen phosphates or sulfates.

10. Process of claims 8 or 9, wherein the acetic acid is added in an amount of 6 to 10 equivalents and the acidic salt in an amount of 20 to 600 equivalents related to 1 equivalent of the oligonucleotide.

11. Process of any one of claims 8 to 10, wherein acetic acid is added.

12. Process of any one of claims 1 to 11, wherein removing of the deprotection by-products in step c) comprises either
c1) extracting the reaction solution with organic solvents and subsequently feeding the solution to tangential flow filtration or
c2) directly feeding the reaction solution to tangential filtration.

13. Process of any one of claims 1 to 12, wherein
a1) acetic acid is added until a pH in the range of pH 11 to 13 is reached;
b1) the reaction mixture is evaporated at a temperature of 40°C to 60°C and at a pressure of 10 mbar to 100 mbar, while removing ammonia until the pH of the solution is in the range of pH 6.0 to pH 8.0;
c1) the resulting reaction mixture is extracted with organic solvents and subsequently fed to tangential flow filtration or,
c2) the resulting reaction mixture is directly fed to tangential flow filtration and optionally
d1) subjected to isolation and further purification.
